# EUROPEAN PATENT APPLICATION

(11) **EP 1 818 410 A1**
(43) Date of publication of application: **15.08.2007**
(21) Application number: 05774958.2
(22) Date of filing: 19.08.2005
(51) Int. Cl.: C12P 7/64

(54) **PROCESS FOR PRODUCING MICROORGANISM FAT CONTAINING DIACYLGLYCEROL IN ANY AMOUNT AND THE FAT**

(30) Priority: 24.08.2004 JP 2004244297
(71) Applicant: SUNTORY LIMITED, Osaka-shi, Osaka 530-8203 (JP)
(72) Inventor: FUJIKAWA, Shigeaki, Takatsuki-shi, Osaka 5691022 (JP); SAKURADANI, Eiji, Uji-shi, Kyoto 6110011 (JP); OGAWA, Jun, Kyoto-shi, Kyoto 6038051 (JP); SHIMIZU, Sakayu, Kyoto-shi, Kyoto 6168212 (JP)
(74) Representative: Vossius, Volker
(86) International application number: PCT/JP2005/015487
(87) International publication number: WO 2006/022356

(57) **Abstract**

In a process for the production of diacylglycerol-containing fat/oil where the ratio of diacylglycerol to the total neutral lipid is more than 20% by weight and a polyunsaturated fatty acid is a constituting fatty acid, a process which is **characterized in that** a microbe being able to produce said fat/oil is incubated and, if desired, said fat/oil is collected.

## Description

### Technical Field

The present invention relates to a process for production of diacylglycerol fat/oil in which a polyunsaturated fatty acid is a constituting fatty acid.

It further relates to a process for production of fat/oil containing a high amount of triacylglycerol and a low amount of diacylglycerol or fat/oil containing a low amount of triacylglycerol and a high amount of diacylglycerol by a fractional distillation of fat/oil which contains triacylglycerol and diacylglycerol.

It still further relates to said fat/oil and to food and drink, to nutritious food for therapy, animal feeds, pet food and to drugs in which said fat/oil is compounded.

In the present invention, the triacylglycerol and the diacylglycerol are those which are extracted from microbes.

### Background Art

The term of a polyunsaturated fatty acid used here stands for a fatty acid having 18 or more carbons and having two or more double bonds. Since a polyunsaturated fatty acid has various unique physiological activities, it is used for the enhancement of functions by adding it to various kinds of foods and animal feeds. Main examples thereof are linoleic acid (LA), α-linolenic acid (ALA), γ-linolenic acid (GLA), dihomo-γ-linolenic acid (DGLA), mead acid (MA), arachidonic acid (AA), eicosapentaenoic acid (EPA) and docosahexaenoic acid (DHA), and so on. In its utilization, although it is used in a free fatty acid type or a phospholipid type, it is mostly used in a triglyceride type and there are many cases where polyunsaturated fatty acid is contained, as a constituting component, in its acyl residues.

In biosynthesis of human polyunsaturated fatty acids, there are two representative types, ω3 type and ω6 type, (ω shows numbers counting from the terminal methyl group of the fatty acid to a carbon where the closest double bond is present) and, in the case of an ω6 type for example, unsaturation and carbon chain extension are repeated from linoleic acid (18:2 ω6) to transform to γ-linolenic acid (18:3 ω6), dihomo-γ-linolenic acid (20:3 ω6), arachidonic acid (20: 4 ω6) and 4, 7, 10, 13, 16-docosapentaenoic acid (22:5 ω6) .

Similarly, in the case of an ω3 type, unsaturation and carbon chain extension are repeated from α-linolenic acid (18:3 ω3) to transform to eicosapentaenoic acid (20 : 5 ω3), 7,10,13,16,19-docosapentaenoic acid (22:5 ω3) and 4,7,10,13,16,19-docosahexaenoic acid (22:6 ω3). Among the polyunsaturated fatty acid of an ω3 type, eicosapentaenoic acid (hereinafter, referred to as "EPA") and docosahexaenoic acid (hereinafter, referred to as "DHA") have been particularly known to have many physiological functions such as preventive effects for adult diseases such as arteriosclerosis and thrombosis, anti-cancer action and enhancing action for learning ability and various attempts have been carried out for their utilization to drugs and designated health foods. In recent years however, attention has been also paid to physiological mechanisms of other types of polyunsaturated fatty acid than the ω3 type (ω6 and ω9 types).

Arachidonic acid occupies about 10% of fatty acids which constitute blood and important organs such as liver (for example, the fatty acid composition ratio of phospholipid of human blood is 11% of arachidonic acid, 1% of eicosapentaenoic acid and 3% of docosahexaenoic acid), participates in adjustment of the fluidity of a membrane as a main constituting component of cell membrane and shows various functions in metabolism in vivo and, on the other hand, it also plays an important role as a direct precursor for prostaglandins. Particularly in recent years, it has been receiving public attention as nutritional supplement for babies and small children as well as for aged people. Usually, when food abundant in linoleic acid is ingested, it is transformed into arachidonic acid but, in patient suffering from adult diseases including people who are about to suffer the diseases, babies/small children and aged people, activities of enzymes participating in biosynthesis lowers and arachidonic acid is apt to become deficient whereby it is desired to directly ingest it as a fat/oil.

As to EPA or DHA which are polyunsaturated fatty acids of an ω3 type, there is an abundant supply source therefor which is fish oil but about γ-linolenic acid, dihomo-γ-linolenic acid, arachidonic acid and 4,7,10,13,16-docosapentaenoic acid (22:5 ω6) which are polyunsaturated fatty acids of an ω6 type, they are rarely available from conventional supplying sources for fat/oil and, at present, fat/oil where polyunsaturated fatty acid prepared by fermentation of microbes is a constituting fatty acid has been generally used. For example, there has been a proposal for a method where various microbes which are able to produce fat/oil in which arachidonic acid is a constituting fatty acid are incubated whereupon fat/oil having arachidonic acid as a constituting fatty acid is produced. Among the above, it has been particularly known that fat/oil containing high amount of arachidonic acid is produced using a microbe of genus *Mortierella* (Japanese Patent Laid-Open No. 63/044,891 and Japanese Patent Laid-Open No. 63/012,290). The fat/oil mainly contains triacylglycerol.

Incidentally, diacylglycerol is also widely present in natural fat/oil. Diacylglycerol has been receiving public attention because it has an action to suppress a rise in neutral fat in blood. Recently, its industrial production by an enzymatic synthetic method has become possible and diacylglycerol has been receiving public attention as food for suppressing a rise in neutral fat in blood and has been utilized as well *(*Kagaku to Kogyo, 74(1), page 33 (2000)). It has been utilized in the field of cosmetics, and drugs, and so on.

Suzuki, et al. (Yukagaku, volume 31, no. 11 (1982), page 921) show that, when *Mortierella isabellina* IFO 7884 is incubated using ammonium carbonate as a nitrogen source, a neutral fat containing 35% of 1,3-diacylglycerol can be produced.

However, a fatty acid composition contained in the diacylglycerol, as such, has not been disclosed.

Further, Suzuki, et al. (Yukagaku, volume 37, no. 11 (1988), page 1081) show that, when dried cells prepared by incubation of *Mortierella isabellina* IFO 8187 is extracted with ethanol which is a polar solvent, diacylglycerol occupying 66.6% of neutral fat is produced. However, no fatty acid composition in the diacylglycerol, as such, is disclosed. In addition, in that method, the polar solvent is also extracted in large quantities and, in order to raise the purity of the neutral lipid, it is further necessary to remove the polar lipid. Moreover, as a result of investigation of extracting conditions, the fat/oil is that where diacylglycerol is concentrated and is not fat/oil *per se* produced by the microbe.

Shimizu, et al. (Biotechnology in Agriculture and Forestry, vol. 33, Medical Aromatic Plants, VIII, page 360) show that a mutant strain of *Mortierella alpine* 1S-4 produces fat/oil containing 20% of diacylglycerol and the amount of arachidonic acid produced therein is 19.4%.

Incidentally, diacylglycerol is also widely present in natural fat/oil. It has been utilized in the field of cosmetics and drugs and so on, moreover, in recent years, a method for producing the same in large quantities and also in high purity has been found, its nutritional study has made a progress. As a result, its utilization as a food for suppressing a rise in neutral fat in blood has been conducted *(*Kagaku to Kogyo, 74 (1) , page 33 (2000) ) .

With regard to a method for an industrial production of diacylglycerol using enzyme or the like, it has been disclosed as follows.

For example, in Japanese Patent Laid-Open No. 01/071,495, diacylglycerol is produced from glycerol and lower alcohol ester of fatty acid using an immobilized lipase whereupon fat/oil where diacylglycerol is 80.1% is produced. Moreover, in Japanese Patent Laid-Open No. 62/025,987, 60 to 70% of diacylglycerol is produced from fatty acid, lower alcohol and glycerol using an alkaline lipase under the condition where no water is substantially present.

However, for the diacylglycerol as such, it is produced using enzymes. Further, no process for production of diacylglycerol containing a polyunsaturated fatty acid has been found yet.

Thus, fat/oil which contains a polyunsaturated fatty acid such as arachidonic acid and further contains diacylglycerol in a high concentration has not been reported yet and it has not been industrially manufactured and its composition has not been known at all. So, its production from the natural world such as microbes without chemical modification of enzyme has been entirely neither known nor carried out.

With regard to microbial fat/oil where triacylglycerol contains arachidonic acid in a high concentration, its production process and the fat/oil per se have been disclosed already but, with regard to microbial fat/oil where amount of triacylglycerol is high while amount of diacylglycerol is low, a production process has not been disclosed yet.

It has been receiving public attention in recent years that diacylglycerol has an action to suppress the rise of neutral fat in blood. However, in view of stability against oxidation, fat/oil containing less monoacylglycerol is preferred. Thus, as compared with a product where diacylglycerol is highly purified, that where triacylglycerol coexists is preferred.

Suzuki, et al. (Yukagaku, volume 37, no. 11 (1988), page 1081) show that diacylglycerol occupying 66.6% of neutral fat is produced but, in the report, dried cells are extracted with ethanol which is a polar solvent and, according to this method, polar lipid is also extracted abundantly whereby it is necessary to further remove the polar lipid in order to improve the purity of the neutral lipid. Moreover, the fat/oil is that where diacylglycerol is concentrated as a result of investigation of extracting condition and is not the fat/oil *per* se which is produced from the microbe.

In Japanese Laid-Open Patents No. 01/071,495 and Nos 62/025,987, the product is produced in an industrial scale using enzymes. Incidentally, no process for production of diacylglycerol containing a highly unsaturated fatty has been found yet.

Shimada, et al. (LIPIDS, vol. 31, no. 12 (2003)) conducted a transesterification reaction between arachidonic acid-containing triacylglycerol and ethanol using an enzyme whereupon, during the reaction, around 25% of diacylglycerol was by-produced but monoacylglycerol was also by-produced to the similar extent.

Thus, a fat/oil which contains a polyunsaturated fatty acid such as arachidonic acid and further contains diacylglycerol in a high concentration has not been reported yet and, nothing to say, it has not been industrially manufactured and its composition has not been known at all. So, its production from natural world such as microbes without chemical modification of enzyme has been entirely neither known nor carried out.

With regard to microbial fat/oil where triacylglycerol contains arachidonic acid in a high concentration, its production process and the fat/oil *per* se have been disclosed already but, with regard to microbial fat/oil where amount of triacylglycerol is high while amount of diacylglycerol is low, its production process has not been disclosed yet.

Although microbial fat/oil containing diacylglycerol has been reported already, the production method therefor is not suitable for an economical production because concentration of diacylglycerol is low. Under such circumstances, it has been aimed to develop a microbe by which fat/oil containing 21% or more diacylglycerol in neutral lipid is able to be produced.
Patent Document 1: Japanese Patent Laid-Open No. 63/044,891
Patent Document 2: Japanese Patent Laid-Open No. 63/012,290
Patent Document 3: Japanese Patent Laid-Open No. 01/071,495
Patent Document 4: Japanese Patent Laid-Open No. 62/025, 987
Non-Patent Document 1: Kagaku to Kogyo, 74(1), page 33 (2000)
Non-Patent Document 2: Yukagaku, volume 31, no. 11 (1982), page 921
Non-Patent Document 3: Yukagaku, volume 37, no. 11 (1988), page 1081
Non-Patent Document 4: Biotechnology in Agriculture and Forestry, vol. 33, Medical Aromatic Plants, VIII, p. 360
Non-Patent Document 5: LIPID, vol. 31, no. 12 (2003)

### Disclosure of the Invention

For the purpose of obtaining a microbe which can produce fat/oil containing 21% or more diacylglycerol in neutral lipids, the present inventors have prepared many mutant strains of *Mortierella alpine* and selected mutant strains which produce fat/oil containing high concentration of diacylglycerol from them. The result was that, to our surprise, when glucose concentration and incubating time are optimized, a process for production of fat/oil in which polyunsaturated fatty acids where diacylglycerol is 30% or more and triacylglycerol is 50% or less to neutral lipid are constituting fatty acids has been achieved.

It is also possible that the fat/oil in the microbial cells is appropriately extracted with a solvent and then separated using a known technique such as fractional distillation whereupon fat/oil containing a higher amount of diacylglycerol and a lower amount of triacylglycerol than the intracellular fat/oil is prepared. One of the fat/oil separated by that method contains a high amount of triacylglycerol and it is possible to prepare fat/oil containing a low concentration of diacylglycerol.

Accordingly, the present invention provides a process for production of fat/oil containing a high amount of diacylglycerol and also provides food and drink, nutritious food for therapy, animal feeds, pet food and drug in which said fat/oil is compounded.

Accordingly, in a process for production of diacylglycerol-containing fat/oil in which polyunsaturated fatty acid is a constituting fatty acid and ratio of diacylglycerol to the total neutral lipid is more than 20% by weight or more, the present invention provides a process which is characterized in that a microbe which is able to produce said fat/oil is incubated and, if desired, said fat/oil is collected therefrom.

In the above-mentioned process, it is preferred that the ratio of diacylglycerol to the total neutral lipid is not less than 30% by weight or, more preferably, the ratio of diacylglycerol to the total neutral lipid is not less than 40% by weight and, for example, the ratio of diacylglycerol to the total neutral lipid is not less than 50% by weight.

In the above-mentioned process, the aforementioned microbe is preferably that belonging to genus *Mortierella* and, more preferably, that belonging to subgenus *Mortierella* of genus *Mortierella* such as a microbe of species *Mortierella alpina.* Preferably, the aforementioned microbe is a variant of a microbe which is able to produce fat/oil containing a polyunsaturated fatty acid.

The present invention also provides a microbe of genus *Mortierella* which is able to produce diacylglycerol-containing fat/oil where a polyunsaturated fatty acid is a constituting fatty acid and the ratio of diacylglycerol to the total neutral lipid is more than 20% by weight or, preferably, a microbe of genus *Mortierella* which is able to produce diacylglycerol-containing fat/oil where a polyunsaturated fatty acid is a constituting fatty acid and the ratio of diacylglycerol to the total neutral lipid is not less than 30% by weight. Preferably, the aforementioned microbe is *Mortierella alpina* and is, for example, a mutant strain thereof.

The present invention further provides microbial cells containing diacylglycerol-containing fat/oil where a polyunsaturated fatty acid is a constituting fatty acid and the ratio of diacylglycerol to the total neutral lipid is more than 20% by weight. Preferably, the ratio of diacylglycerol to the total neutral lipid is not less than 30% by weight and, more preferably, the ratio of diacylglycerol to the total neutral lipid is not less than 40% by weight. For example, the ratio of diacylglycerol to the total neutral lipid is not less than 50% by weight. The above-mentioned microbe is preferably microbial cells of a microbe belonging to genus *Mortierella* and, for example, it is microbial cells of a microbe of species *Mortierella alpina.* Microbial cells are sterilized, living or dried.

The present invention still further provides diacylglycerol-containing fat/oil where a polyunsaturated fatty acid is a constituting fatty acid and the ratio of diacylglycerol to the total neutral lipid is more than 20% by weight where said fat/oil is extracted with incubated cells of a microbe which is able to produce said fat/oil. Preferably, the ratio of diacylglycerol to the total neutral lipid is not less than 30% by weight and, more preferably, the ratio of diacylglycerol to the total neutral lipid is not less than 40% by weight. For example, the ratio of diacylglycerol to the total neutral lipid is not less than 60% by weight. For example, the above-mentioned extraction is carried out using a nonpolar solvent or a hydrophilic solvent.

The present invention furthermore provides fat/oil containing not less than 70% of diacylglycerol and not more than 30% of triacylglycerol in a neutral lipid prepared by fractionation by extraction and distillation of fat/oil from cells containing diacylglycerol and triacylglycerol prepared by incubation of a microbe which is able to produce fat/oil comprising a polyunsaturated fatty acid as a constituting fatty acid.

The present invention still furthermore provides fat/oil containing not less than 95% of triacylglyceride and not more than 5% of diacylglycerol in a neutral lipid prepared by fractionation by extraction and distillation of fat/oil from cells containing diacylglycerol and triacylglycerol prepared by incubation of a microbe which is able to produce fat/oil comprising a polyunsaturated fatty acid as a constituting fatty acid.

The polyunsaturated fatty acid constituting the fat/oil of the present invention is, for example, dihomo-γ-linolenic acid (20:3, ω6) , arachidonic acid (20:4 ω6), 7, 10, 13, 16-docosatetraenoic acid (22:4 ω6), 4,7,10,13,16-docosapentaenoic acid (22:5 ω6), 6,9,12,15-octadecatetraenoic acid (18:4 ω3), 8,11,14,17-eicosatetraenoic acid (20:4 ω3), eicosapentaenoic acid (20:5 ω3), 7, 10, 13, 16, 19-docosapentaenoic acid (22:5 ω3), 4, 17, 10, 13, 16, 19-docosahexaenoic acid (22:6 ω3), 6,9-octadecadienoic acid (18:2 ω9) , 8, 11-eicosadienoic acid (20:2 ω9) or 5, 8, 11-eicosatrienoic acid (mead acid: 20:3 ω9) or a combination thereof.

The present invention also provides food composition, animal feeds composition, pet food, materials for chemicals, drug and cosmetic composition where the above-mentioned fat/oil of the present invention is contained or the fat/oil is used as a material or is chemically modified.

### Best Mode for Carrying Out the Invention

### Preparation of mutant strain

The present invention relates to food and drink, nutritious food for therapy and drugs compounded with pure fat/oil which is prepared in such a manner that a microbe which is able to produce fat/oil in which amount of a polyunsaturated fatty acid where diacylglycerol in fat/oil, specifically in crude oil, is high as a constituting fatty acid is incubated to produce said fat/oil and then said fat/oil is purified.

Accordingly, in the present invention, any microbe is able to be used so far as it is a microbe which is able to produce fat/oil (diacylglycerol) where a polyunsaturated fatty acid is a constituting fatty acid. For example, with regard to a microbe which is able to produce fat/oil (triglyceride) where arachidonic acid is a constituting fatty acid, microbes belonging to genus *Mortierella*, genus Conidiobolus, genus Pythium, genus *Phytophthora,* genus *Penicillium,* genus *Cladosporium,* genus Mucor, genus *Fusarium,* genus Aspergillus, genus *Rhodotorula,* genus *Entomophthora,* genus *Echinosporansium* and genus *Saprolegnia* may be listed.

With regard to a microbe belonging to genus *Mortierella* and subgenus *Mortierella*, examples thereof are *Mortierella elongata*, *Mortierella* exigua, *Mortierella hygrophila* and *Mortierella alpina,* and so on.

Specific examples thereof are strains of *Mortierella elongata* IFO 8570, *Mortierella exigua* IFO 8571, *Mortierella hygrophila* IFO 05941, *Mortierella alpina* IFO 8568, ATCC 16266, ATCC 32221, ATCC 42430, CBS 291.35, CBS 224.37, CBS 250.53, CBS 343.66, CBS 527.72, CBS 529.72, CBS 608.70 and CBS 754.68.

For example, with regard to a microbe which is able to produce DHA, a microbe belonging to genus *Crypthecodenium,* genus *Thrautochytrium,* genus *Schizochytrium,* genus Ulkenia, genus *Japonochytrium* or genus *Haliphthoros* may be listed.

All of those strains are available without any restriction from the Institute for Fermentation, Osaka (IFO), the American Type Culture Collection (ATCC), U. S. A. and the Centraalbureau voor Schimmelcultures (CBS). It is also possible to use *Mortierella elongata* SAM 0219 (Accession Number 8703 at the Fermentation Research Institute) (Accession Number 1239 at the Fermentation Research Institute according to the Treaty) which is a strain separated by the study group of the present invention from the soil.

Although the strains belonging to those type cultures or the strains separated from nature may be used as they are, it is also possible to use a natural mutant prepared by one or more growth and/or separation operation(s) and having different properties from those of the original strains. Thus, when the lipid-producing microbe is subjected to a mutation treatment and selected, it is also possible to select a lipid-productive microbe having an enhanced productivity of diacylglycerol.

Although there is no particular limitation for the mutation treatment so far as it is applicable to the above-mentioned lipid-producing microbe, an example is a common mutation treatment such as irradiation with radioactive ray (X-ray, gamma-ray and neutron ray), irradiation of ultraviolet ray, treatment at high temperature and a method where a microbe is suspended in an appropriate buffer or the like, mutagen is added thereto, the mixture is incubated for a predetermined period, appropriately diluted and inoculated to an agar medium to give colonies of a mutant strain. Examples of the mutagen are alkylating agents such as nitrogen mustard, methylmethane sulfonate and N-methyl-N'-nitro-N-nitrosoguanidine (NTG); base analogs such as 5-bromouracil; antibiotics such as mitomycin C; inhibitors for base synthesis such as 6-mercaptopurine; dyes such as proflavine; certain types of cancer-causing agent such as 4-nitroquinoline-N-oxide; and compounds such as manganese chloride and formaldehyde. The microbe used may be either growing cells (hyphae) or spores.

Although there is no particular limitation for a method by which a mutated lipid-producing microbe having an enhanced productivity for diacylglycerol is selected a mutated lipid-producing microbe, it is preferred that lipid which is produced by a mutated lipid-productive microbe is analyzed by a high-performance liquid chromatography or the like.

As shown in Example 1, when about 3,000 cells of the strain subjected to mutation were selected as mentioned above, three strains of mutants which produce diacylglycerol-containing fat/oil where the ratio of diacylglycerol to the total neutral lipid is more than 20% by weight and a polyunsaturated fatty acid is a constituting fatty acid were obtained. That means one strain in average of aimed mutant strain was obtained per 1,000 mutated strains. Accordingly, frequency of preparation of the mutant strain of the present invention is far higher than the case of a random selection by conventional mutation and selection and the mutant strain having the above-mentioned characteristic similar to the three strain mutants actually prepared in the present invention is able to be easily prepared by repeating the method mentioned in Example 1 of the present invention.

A microbe belonging to genus *Mortierella* and subgenus *Mortierella* has been known as a microbe which is able to produce fat/oil (triacylglycerol) where arachidonic acid is a main constituting fatty acid and, as a result of subjecting the above-mentioned strain to a mutation treatment, the present inventors have prepared a microbe which is able to produce fat/oil (triacylglyceride) where dihomo-γ-linoleic acid is a main constituting fatty acid (Japanese Patent Laid-Open No. 05/091,887) and a microbe which is able to produced fat/oil (triacylglyceride) where an ω6 type polyunsaturated fatty acid is a main constituting fatty acid (Japanese Patent Laid-Open No. 05/091,888). They have also prepared a microbe having a resistance to carbon source in a high concentration (WO 98/39468). Those microbes are microbes belonging to genus *Mortierella* and subgenus *Mortierella* and, when those strains are subjected to the same mutating treatment as in the present invention, it is possible to prepare microbes which accumulate fat/oil having a high amount of diacylglycerol where dihomo-γ-linolenic acid or ω9 type polyunsaturated fatty acid as a main constituting fatty acid.

The fat/oil of the present invention is a microbial fat/oil which is prepared from an incubated product obtained by incubation of a microbe which is able to produce fat/oil where a polyunsaturated fatty acid is a constituting fatty acid. Thus, as a result of incubation of a microbe in an incubation tank, fat/oil containing a high concentration of diacylglycerol in the cells is accumulated and then said fat/oil is extracted therefrom.

To be more specific, it is a fat/oil containing not less than 21% by weight or, preferably, not less than 35% by weight of diacylglycerol to the fat/oil and containing not less than 2% by weight or, preferably, not less than 9% by weight of a polyunsaturated fatty acid to the total fatty acids in diacylglycerol. Accordingly, it is essential to incubate a microbe which is able to produce a fat/oil (diacylglycerol) where a polyunsaturated fatty acid is a constituting fatty acid. With regard to the microbe used here, it is preferrably a microbe which produces at least one of an ω6 type polyunsaturated fatty acid where carbon numbers are 18 or more and double bonds are 3 or more, an ω9 type polyunsaturated fatty acid where carbon numbers are 18 or more and double bonds are 2 or more and an ω3 type polyunsaturated fatty acid where carbon numbers are 18 or more and double bonds are 3 or more as a main constituting fatty acid for diacylglycerol.

With regard to the ω6 type polyunsaturated fatty acid where carbon numbers are 18 or more and double bonds are 3 or more, examples thereof are γ-linolenic acid (6,9,12-octadecatrienoic acid), dihomo-γ-linolenic acid (8,11,14-eicosatrienoic acid), arachidonic acid (5,8,11,14-eicosatetraenoic acid), 7,10,13,16-docosatetraenoic acid (22:4 w6) and DPA ω6 (4,7,10,13,16-docosapentaenoic acid); with regard to the ω9 type polyunsaturated fatty acid where carbon numbers are 18 or more and double bonds are 2 or more, examples thereof are 6,9-octadecadienoic acid, 8,11-eicosadienoic acid and mead acid (5, 8, 11-eicosatrienoic acid); and, with regard to the ω3 type polyunsaturated fatty acid where carbon numbers are 18 or more and double bonds are 3 or more, examples thereof are α-linolenic acid (9,12,15-octadecatrienoic acid), 6, 9, 12, 15-octadecatetraenoic acid (18:4 ω3), 8, 11, 14, 17-eicosatetraenoic acid (20:4 ω3) , EPA (5,8,11,14,17-eicosapentaenoic acid), DPA ω3 (7, 10, 13, 16, 19-docosapentaenoic acid) and DHA (4, 7, 10, 13, 16, 19-docosahexaenoic acid). In order to incubate the strain used in the present invention, spores or mycelia of said strain or a seed culture liquid prepared by a previous incubation or cells recovered from the seed culture is inoculated to a liquid medium to conduct a main incubation. In the liquid medium, as a carbon source, any of commonly used ones such as glucose, fructose, xylose, saccharose, maltose, soluble starch, molasses, glycerol, mannitol and saccharified starch may be used although they are non-limitative. With regard to a nitrogen source, an organic nitrogen source such as urea and an inorganic nitrogen source such as sodium nitrate, ammonium nitrate and ammonium sulfate are able to be used besides a natural nitrogen source such as peptone, yeast extract, malt extract, meat extract, Casamino acid, corn steep liquor, soybean protein, defatted soybean and cotton seed cake.

With regard to a nitrogen source prepared from soybean, specific examples thereof are soybean, defatted soybean, soybean flakes, edible soybean protein, bean-curd refuse, soybean milk and soybean powder and, particularly, a product prepared by subjecting the defatted soybean to a heating denaturation or, more preferably, a product prepared by subjecting the defatted soybean to a heating treatment at about 70 to 90°C followed by removing of ethanol-soluble components therefrom may be used either solely or jointly or in combination with the aforementioned nitrogen source. Moreover, if necessary, in addition to phosphate ion, potassium ion, sodium ion, magnesium ion and calcium ion, it is also possible to use metal ion such as iron, copper, zinc, manganese, nickel and cobalt, vitamin and the like as minor nutrients.

There is no particular limitation for the medium component as such so far as it is within a concentration which does not disturb the growth of the microbe. Practically, it is usually desirable that the total adding amount of the carbon source is 0.1 to 40% by weight or, preferably, 1 to 25% by weight and that the total adding amount of the nitrogen source is 1 to 15% by weight or, preferably, 2 to 10% by weight. More preferably, the incubation is carried out under such a condition that the initial adding amount of the carbon source is 1 to 5% by weight and the initial adding amount of the nitrogen source is 3 to 8% by weight and that, during the incubation, the carbon source and the nitrogen source or, still more preferably, only a carbon source are/is added thereto followed by incubating.

In order to increase the yield of the polyunsaturated fatty acid, as a precursor for the polyunsaturated fatty acid, it is possible to use, for example, a hydrocarbon such as hexadecane or octadecane; fatty acid such as oleic acid or linoleic acid or a salt thereof or fatty acid ester such as ethyl ester, glycerol fatty acid ester or sorbitan fatty acid ester; or fat/oil such as olive oil, soybean oil, rapeseed oil, cottonseed oil or coconut oil either solely or in combination thereof. Adding amount of the substrate to the medium is 0.001 to 10% or, preferably, 0.5 to 10%. It is also possible that such a substrate is used as a sole carbon source.

Although the incubating temperature for the microbe which produces a polyunsaturated fatty acid varies depending upon the type of the microbe used, it is 5 to 40°C or, preferably, 20 to 30°C or it is also possible that incubation is conducted at 20 to 30°C to proliferate the cells and, after that, the incubation is continued at 5 to 20°C whereupon the polyunsaturated fatty acid is produced. Even by temperature control, as such, it is still possible to raise the ratio of the polyunsaturated fatty acid in the resulting fatty acids. In a seed culture, incubation under aeration with stirring, shake culture, solid culture or liquid culture under being allowed to stand are conducted while, in a main culture, incubation under aeration with stirring is conducted. Incubating period is usually 2 to 30 days, preferably 5 to 20 days and, more preferably, 5 to 15 days.

For example, a diacylglycerol acid-productive KY-1 strain and 4,000 L of 2% yeast extract and 6% of glycerol (pH 6.0) are placed in a 10-kL incubator for aeration with stirring and subjected to incubation with aeration and stirring for 7 days under the condition where temperature was 28°C, aerating amount was 1 VVM and inner pressure of the incubator was 1.0 kg/cm² , then sterilization was conducted after completion of incubation and wet cells were recovered using a continuous drying machine and dried using a drier until the water content reached 5 wt% to give dry cells.

The fat/oil of the present invention is a microbial fat/oil where a microbe which is able to produce a fat/oil (diacylglycerol) in which a polyunsaturated fatty acid is a constituting fatty acid is incubated and is prepared from the incubated product and its biggest characteristic is that incubation is conducted using an incubating vessel whereupon amount of diacylglycerol in fat/oil contained in the cells is increased. Therefore, incubating medium, incubating condition and method for extraction and purification of fat/oil are not limited to the aforementioned ones.

### Recovery of cells and extraction of fat/oil

With regard to a process for the production of fat/oil from a microbe in which fat/oil where a polyunsaturated fatty acid containing high amount of diacylglycerol is a constituting fatty acid is accumulated in the cells, incubated cells are prepared by a commonly used solid-liquid separating means such as natural sedimentation, centrifugal separation and/or filtration for the culture liquid after completion of the incubation as it is or after subjecting to a treatment such as sterilization, concentration or acidification. In order to assist the solid-liquid separation, a coagulant or a filtering aid may be added as well. Examples of the coagulant which may be used are aluminum chloride, calcium chloride, alginate and chitosan, and so on. Examples of the filtering aid which may be used are diatomaceous earth, etc. Preferably, the incubated cells are washed with water, disintegrated and dried.

Drying may be conducted by freeze-drying, airdrying, drying with a fluidized bed, etc. Although extraction with an organic solvent or compression may be used as a means for the preparation of crude oil from dry cells, it is preferred to extract using an organic solvent in a nitrogen stream. With regard to the organic solvent, ethanol, hexane, methanol, ethanol, chloroform, dichloromethane, petroleum ether, acetone, etc. may be used. It is also possible to conduct an alternate extraction using methanol and petroleum or to use a three-layer type solvent comprising chloroform, methanol and water.

Among those solvents, when a hydrophobic solvent such as hexane is used for the extraction, a neutral lipid such as diacylglycerol and triacylglycerol can be extracted with high purity without extracting a polar lipid such as phospholipid. The resulting extracted fat/oil is able to give pure fat/oil without causing a big load on the purification steps, such as a degumming step for removal of phospholipid or the like and a deacidifying step for removal of free fatty acid or the like.

When a hydrophilic solvent such as ethanol is further used, although a polar lipid such as phospholipid is extracted, diacylglycerol is able to be extracted more selectively whereupon it is possible to extract a diacylglycerol fat/oil in a higher concentration than a diacylglycerol concentration in the neutral fat/oil existing in the cells. For example, when ethanol is used as a solvent, it is possible to prepare a fat/oil containing not less than 70% of diacylglycerol and not more than 30% of triacylglycerol in a neutral lipid.

When the residue after extraction with ethanol is further extracted with hexane, triacylglycerol concentration existing in the cells is able to be enhanced. Thus, it is possible to prepare a fat/oil containing not more than 10% of diacylglycerol and not less than 80% of triacylglycerol in neutral lipid.

Method for extraction of crude oil is not limited to the above-mentioned ones but any means by which fat/oil in microbial cells is efficiently extracted is able to be used. For example, a supercritical extraction method, etc. may be used as an effective means.

The aimed crude oil can be prepared by removal of an organic solvent and a supercritical fluid component under the condition such as *in vacuo* from an extract which is extracted with the organic solvent or the supercritical fluid. Alternatively, it is possible to conduct an extraction using wet microbial cells instead of the above-mentioned methods. In that case, a solvent which is miscible with water such as methanol, ethanol, acetone, etc. or a water-miscible mixed solvent comprising the above and water and/or other solvent is used. Other procedures are the same as above.

The crude oil prepared by the present invention where a polyunsaturated fatty acid in which diacylglycerol amount is increased is a constituting fatty acid is able to be directly used by compounding with feeds for animals. However, when adaptability thereof to food is taken into consideration, it is desirable to use after subjecting to a common purifying step for fat/oil. With regard to the purifying step for a fat/oil, commonly used ones such as degumming, deacidifying, deodorizing, decolorizing, treatment with column, molecular distillation and wintering may be used.

When a molecular distillation is used under an appropriate condition, a highly pure diacylglycerol-containing pure fat/oil which has not been available up to now is able to be prepared in a distilled fat/oil fraction. Moreover, a fat/oil prepared by such a manner that fat/oil containing a high concentration of triacylglycerol as a result of extraction with hexane is further subjected to a molecular distillation whereupon diacylglycerol is removed as a distillate is able to be prepared as a fat/oil containing triglyceride of as high as not less than 90% and diacylglycerol of as low as not more than 5%.

Thus, when a fat/oil containing a high concentration of diacylglycerol is further subjected to various extracting and purifying methods, it is possible to prepare a fat/oil in which diacylglycerol and triacylglycerol are in any purity depending on the aimed use.

### Use of the fat/oil

With regard to the use of the fat/oil (diacylglycerol and triacylglycerol), there are unlimited possibilities and that is able to be used as a material for and an additive to foods, drinks, animal feeds, pet food, cosmetics and drugs. Particularly, utilization in foods, utilizing the fact that diacylglycerol has higher hydrophilicity than triacylglycerol, is greatly widened.

For example, with regard to a food composition, besides common foods, there are listed functional foods, nutritious supplements, processed milk for premature babies, processed milk for matured babies, processed milk for babies, foods for babies, foods for pregnant women and nursing mothers and foods for aged people. Examples of the fat/oil-containing foods are natural food containing the inherent fat/oil such as meat, fish or nuts; food such as soup where fat/oil is added upon cooking; food such as doughnut where fat/oil is used as a heat medium; fat/oil food such as butter; processed food such as cookie where fat/oil is added upon processing; and food such as hard biscuit where fat/oil is sprayed or applied upon finish of processing. It is also possible to add to agricultural food, fermented food, stock farm food, fishery food or drink which does not contain fat/oil. It may also be in a form of a functional food and a drug and, for example, processed form such as enteral nutrient, powder, granule, troche, peroral liquid, suspension, emulsion and syrup may be acceptable.

### Examples

The present invention will now be more specifically illustrated by way of the following Examples although the present invention is not limited to those Examples.

### Example 1: Preparation of mutant strain

*M. alpina* 1S-4 was inoculated in a big slant bottle containing 300 ml of a Czapek's agar medium (0.2% NaNO₃, 0.1% K₂HPO₄, 0.05% MgSO₄.7H₂O, 0.05% KCl, 0.01% FeSO₄.7H₂O, 3% sucrose and 2% agar; pH 6.0) and incubated for 2 weeks at 28°C to form spores. After the incubation, 50 ml of aseptic water to which two drops of Tween 80 was added was added to the big slant bottle, well shaken and filtered through four gauzes. Such an operation was repeated twice and the filtrate was centrifuged at 8,000 x g for 10 minutes.

The spores prepared as such were suspended in a Tris maleate buffer (50 mM Tris, 50 mM maleate; pH 7.5) to produce 1 × 10⁶ spores/ml. After that, a mutation treatment was carried out using NTG.

The spore suspension treated with NTG was diluted to several concentration stages and applied to a GY agar medium (1% glucose, 0.5% yeast extract, 0.005% Triton X-100 and 1.5% agar; pH 6.0). Incubation was conducted at 28°C and, as from a product where colonies appeared, it was randomly picked up on a new plate. After formation of colonies of 0.5 to 1 cm at 28°C, incubation was conducted at 12°C for 2 days.

The mutant strain picked up to the plate as such was subjected to a shake culture for 7 days in a test tube (12.3 x 200 mm) containing 4 mm of GY liquid medium (2% glucose and 1% yeast extract; pH 6.0). Lipid in the resulting cells was extracted according to the following Bligh-Dyer method. Thus, about 1 g of the cells were placed in a mortar, well ground down with 2 ml of 1% aqueous solution of KCl and transferred to a screwed test tube. Then 4 ml of a solvent A (CHC1₃/MeOH, 2/1, v/v) was added thereto, the mixture was subjected to a rotational stirring so that the two layers were well mixed and centrifuged for several minutes and the lower CHCl₃ layer was collected. The resulting CHCl₃ layer separated as such was concentrated to dryness using a centrifugal evaporator, around 500 µl of the solvent A was added thereto and the mixture was stored at -20°C.

In the TLC, a plate (Art 5751; 200 x 200 x 0.25 mm; Merck) coated with silica gel was used. Hexane/diethyl ether/acetic acid (70:30:1, v/v) was used as a developing solvent so that triacylglycerol (TG), diacylglycerol (DG), monoacylglycerol (MG) and free fatty acid (FFA) were mainly analyzed. After the developing, a 5% (w/v) ethanolic solution of phosphomolybdenic acid was sprayed followed by heating at 120°C to detect the spots of each of the lipids and the spots where the mode was different from a parent strain were searched.

As a result, a strain where, in the first screening in the TLC, the spot of triacylglycerol (TG) was lighter or the spots of diacylglycerol (DG) and free fatty acid (FFA) were darker than spots of other lipid components was confirmed and the strain was subjected to an HPLC analysis using an evaporation light scattering analysis system, it has been clarified that contained amounts of diacylglycerol and sterol were somewhat high as a result of comparison with the standard (1S-4) which is a parent strain.

As a result of checking of about 3,000 colonies, three mutant strains (#1 strain, #2 strain and #3 strain), in which the ratio of diacylglycerol in the total lipid was enhanced, were obtained. Contents of diacylglycerol in those strains were 31%, 28% and 22%, respectively.

**Table 1**

| | Diacylglycerol | Triacylglycerol | Monoacylglycerol | Others |
|---|---|---|---|---|
| #1 | 31% | 65% | N.D. | 5% |
| #2 | 28% | 68% | N.D. | 4% |
| #3 | 22% | 73% | N.D. | 5% |

| | | | | |
|---|---|---|---|---|
| N.D.: not detected | | | | |

### Example 2: Comparison of Mutant Strain Prepared in Example 1 with the Parent Strain

Among the three mutant strains (#1 strain, #2 strain and #3 strain) where the ratio of diacylglycerol was enhanced, the #1 strain (KY-1 strain) where the amount of diacylglycerol was the highest was inoculated to a medium containing 1% of yeast extract and 0, 2, 3, 4 or 6% of glucose, incubation was initiated under the condition of a reciprocating shaking of 300 rpm and temperature of 28°C, and incubation was conducted for 7 days.

Amounts of triacylglycerol, diacylglycerol and sterol per wet cells after completion of the incubation were analyzed by a method of Example 1 and the result thereof is shown in Table 2. No monoacylglycerol was detected. In the incubation where glucose was 2%, diacylglycerol in the cells was as highest as 15.36 mg/g and the ratio of diacylglycerol in the neutral lipid was also as high as 34.5%.

**Table 2**

| Glucose Concn (%) | Wet Cell Weight (mg/ml culture liquid) | Triacylglycerol (mg/g wet cells) | Sterol (mg/g wet cells) | 1,2-Diacylglycerol (mg/g wet cells) |
|---|---|---|---|---|
| 0 | 1.1 | 1.09 | 2.65 | 0 |
| 2 | 7.7 | 11.79 | 17.35 | 15.36 |
| 3 | 11.8 | 11.33 | 0.54 | 5.41 |
| 4 | 12.6 | 8.21 | 0.39 | 2.14 |
| 6 | 13.8 | 11.67 | 0.15 | 1.15 |

As a comparative example, the 1S-4 strain was incubated by the same method as in the Example and analysis was conducted. The result is shown in Table 3.

**Table 3**

| Glucose Concn (%) | Wet Cell Weight (mg/ml culture liquid) | Triacylglycerol (mg/g wet cells) | Sterol (mg/g wet cells) | 1,2-Diacylglycerol (mg/g wet cells) |
|---|---|---|---|---|
| 0 | 2.9 | 2.16 | 0 | 0 |
| 2 | 12.2 | 18.83 | 0.60 | 0.59 |
| 3 | 13.5 | 16.23 | 1.59 | 0 |
| 4 | 14.3 | 8.17 | 1.25 | 0 |
| 6 | 13.9 | 28.33 | 0.18 | 0 |

### Example 3

KY-1 strain which is a diacylglycerol acid-productive microbe was inoculated to 4 ml GY medium (1% yeast extract and 2% glucose; pH 6.0) placed in a 100-ml Erlenmeyer flask and started to incubation under the condition where shaking was 300 rpm and temperature was 28°C and the incubation was conducted for 4, 6 or 8 days.

The cells after completion of the incubation were subjected to a measurement of weight of the cells after drying. Lipid was extracted from the cells prepared as such by Bligh-Dyer method and quantification of each lipid component was conducted using an evaporation light scattering detector of liquid chromatography.

The result is shown in Table 4. No monoacylglycerol was detected. On the eighth day of the incubation, total neutral lipid mass per cell was as highest as 16 mg/g. Ratios of the diacylglycerol in the total neutral lipid on fourth, sixth and eighth days of the incubation were 13%, 31.5% and 18.1%, respectively.

**Table 4**

| Days for Incubation | Amount of Lipids (mg/g cells) | | | | |
|---|---|---|---|---|---|
| | Total | Triacylglycerol | Diacylglycerol | Free Fatty Acid | Ratio of Diacylglyce rol (%) |
| 4 days | 1.5 | 8.6 | 1.5 | 1.4 | 13.0 |
| 6 days | 13.0 | 5.9 | 4.1 | 3.0 | 31.5 |
| 8 days | 16.0 | 9.5 | 2.9 | 3.6 | 18.1 |

The result where fatty acid compositions contained in triacylglycerol, diacylglycerol and free fatty acid on fourth, sixth and eighth days of the incubation were analyzed is shown in Table 5. Arachidonic acid was contained in an amount of 8.1 to 13.8% in diacylglycerol.

**Table 5**

| Fatty Acid Name | Common Name | 4th day | | | 6th day | | | 8th day | | |
|---|---|---|---|---|---|---|---|---|---|---|
| | | FAC in TG (%) | FAC in DG(%) | FAC in FA (%) | FAC in TG (%) | FAC in DG (%) | FAC in FA (%) | FAC in TG(%) | FAC in DG(%) | FAC in FA (%) |
| C14:0 | Myristic Acid | 2.5 | 1.1 | 4.2 | 2.1 | 1.1. | 1.4 | 1.6 | 1.0 | 1.2 |
| C15:0 | | 11.6 | 7.9 | 11.4 | 8.1 | 6.6 | 6.0 | 7.3 | 6.1 | 5.5 |
| C16:0 | Palmitic Acid | 16.6 | 10.0 | 14.9 | 22.8 | 15.0 | 15.5 | 22.1 | 15.9 | 18.0 |
| C17:0 | | 10.7 | 7.5 | 19.3 | 8.3 | 4.2 | 3.7 | 8.1 | 4.3 | 4.8 |
| C17:1 | | 11.7 | 13.4 | 7.2 | 7.6 | 7.6 | 5.9 | 7.3 | 7.9 | 6.3 |
| C18:0 | Stearic Acid | 5.7 | 4.3 | 7.5 | 4.2 | 2.5 | 2.1 | 10.6 | 2.4 | 3.0 |
| C18:1 | Oleic Acid | 24.8 | 33.2 | 18.2 | 24.4 | 28.0 | 29.4 | 23.3 | 30.0 | 28.4 |
| C18:2 | Linoleic Acid | 3.7 | 5.9 | 4.0 | 4.9 | 11.8 | 12.1 | 4.5 | 11.3 | 11.6 |
| C18:3 | γ-Linolenic Acid | 3.5 | 5.7 | 3.1 | 4.0 | 5.7 | 3.7 | 3.2 | 5.7 | 3.7 |
| C20:0 | Arachidic Acid | 0.7 | 0.0 | 0.7 | 0.5 | 0.3 | 0.5 | 0.5 | 0.2 | 0.8 |
| C20:1 | | 0.5 | 0.3 | 0.6 | 0.5 | 0.5 | 0.4 | 0.5 | 0.6 | 0.4 |
| C20:3 | Dihomo-γ-linolenic Acid | 2.0 | 2.1 | 0.8 | 2.4 | 2.3 | 2.4 | 2.3 | 2.2 | 2.1 |
| C20:4 | Arachidonic Acid | 5.1 | 8.1 | 4.6 | 8.5 | 13.8 | 12.6 | 6.7 | 11.9 | 6.6 |
| C22:0 | Behanic Acid | 0.1 | 0.0 | 1.2 | 0.4 | 0.0 | 1.2 | 0.4 | 0.0 | 2.4 |
| C24:0 | | 0.8 | 0.3 | 2.2 | 1.2 | 0.6 | 3.2 | 1.5 | 0.5 | 5.2 |

| | | | | | | | | | | |
|---|---|---|---|---|---|---|---|---|---|---|
| FFC: Fatty acid composition TG: Triacylglycerol DG: Diacylglycerol FA: Free fatty acid | | | | | | | | | | |

As a comparative example, the 1S-4 strain was incubated by the same method as in the Example and analysis was conducted. The result is shown in Table 6. On the sixth day of the incubation, accumulated amount of diacylglycerol was as highest as 2.4 mg/g cells and, at that time, the ratio of diacylglycerol to the neutral lipid was as small as 5.8%.

**Table 6**

| Days for Incubation | Amount of Lipids (mg/g cells) | | | | |
|---|---|---|---|---|---|
| | Total | Triacylglycerol | Diacylglycerol | Free Fatty Acid | Ratio of Diacylglycerol (%) |
| 4 days | 70.9 | 64.9 | 2.4 | 3.6 | 3.4 |
| 6 days | 41.1 | 34.8 | 2.4 | 3.9 | 5.8 |
| 8 days | 17.1 | 14.7 | 1.0 | 1.4 | 5.6 |

### Example 4: Manufacture by an Incubation in 50-L Jar Fermenter

KY-1 strain which is a diacylglycerol acid-productive microbe was inoculated to a 50-L vessel for incubation by aeration with stirring containing 30 L of a medium (pH 6.0) containing 2% of yeast extract and 2% of glucose and subjected to an incubation by aeration with stirring under the condition where temperature was 28°C, aerating amount was 1 VVM and stirring was 200 rpm. Glucose was added thereto so that glucose concentration increased to an extent of 2% on the second, third and fourth days of the incubation. After completion of the incubation, sterilization was conducted under the condition of 98°C for 20 minutes and wet cells were recovered by filtration and dried using a drying machine until water content became 5 wt% to give 630 g of dried cells. Fat/oil was extracted from the cells using hexane to give 43 g of fat/oil.

Diacylglycerol contained in neutral lipid of this fat/oil was 36% and arachidonic acid contained in the diacylglycerol was 15.2% of the total fatty acid.

## Claims

1. A process for production of diacylglycerol-containing fat/oil where the ratio of diacylglycerol to the total neutral lipid is more than 20% by weight and a polyunsaturated fatty acid is a constituting fatty acid, the process being **characterized in that** a microbe being able to produce said fat/oil is incubated and, if desired, said fat/oil is collected.

2. The process according to claim 1, wherein the ratio of diacylglycerol to the total neutral lipid is not less than 30% by weight.

3. The process according to claim 1, wherein the ratio of diacylglycerol to the total neutral lipid is not less than 40% by weight.

4. The process according to claim 1, wherein the ratio of diacylglycerol to the total neutral lipid is not less than 50% by weight.

5. The process according to any of claims 1 to 4, wherein the microbe is a microbe of genus *Mortierella.*

6. The process according to claim 5, wherein the microbe is a microbe of genus *Mortierella,* subgenus *Mortierella.*

7. The process according to claim 6, wherein the microbe is a microbe of species *Mortierella alpina.*

8. The process according to any of claims 5 to 7, wherein the microbe is a mutant strain of a microbe which is able to produce fat/oil containing a polyunsaturated fatty acid.

9. A microbe of genus *Mortierella* which is able to produce diacylglycerol-containing fat/oil where the ratio of diacylglycerol to the total neutral lipid is not less than 20% by weight and a polyunsaturated fatty acid is a constituting fatty acid.

10. A microbe of genus *Mortierella* which is able to produce diacylglycerol-containing fat/oil where the ratio of diacylglycerol to the total neutral lipid is not less than 30% by weight and a polyunsaturated fatty acid is a constituting fatty acid.

11. The microbe according to claim 9 or 10, wherein it is *Mortierella alpina.*

12. The microbe according to any of claims 9 to 11, wherein it is a mutant strain.

13. A microbial cell containing diacylglycerol-containing fat/oil where the ratio of diacylglycerol to the total neutral lipid is more than 20% by weight and a polyunsaturated fatty acid is a constituting fatty acid.

14. The microbial cell according to claim 13, wherein the ratio of diacylglycerol to the total neutral lipid is not less than 30% by weight.

15. The microbial cell according to claim 13, wherein the ratio of diacylglycerol to the total neutral lipid is not less than 40% by weight.

16. The microbial cell according to claim 13, wherein the ratio of diacylglycerol to the total neutral lipid is not less than 50% by weight.

17. The microbial cell according to any of claims 13 to 16, wherein the microbe is a microbe of genus *Mortierella.*

18. The microbial cell according to claim 17, wherein the microbe is a microbe of species *Mortierella alpina.*

19. Fat/oil which is extracted from incubated cells of a microbe being able to produce the fat/oil where the ratio of diacylglycerol to the total neutral lipid is more than 20% by weight and a polyunsaturated fatty acid is a constituting fatty acid.

20. The fat/oil according to claim 19, wherein the ratio of diacylglycerol to the total neutral lipid is not less than 30% by weight.

21. The fat/oil according to claim 19, wherein the ratio of diacylglycerol to the total neutral lipid is not less than 40% by weight.

22. The fat/oil according to claim 19, wherein the ratio of diacylglycerol to the total neutral lipid is not less than 60% by weight.

23. The fat/oil according to any of claims 19 to 22, wherein it is extracted with a nonpolar solvent.

24. The fat/oil according to any of claims 19 to 22, wherein it is extracted with a hydrophilic solvent.

25. A fat/oil in which diacylglycerol and triacylglycerol in a neutral lipid are not less than 70% and not more than 30%, respectively which is prepared in such a manner that fat/oil is extracted from microbial cells containing diacylglycerol and triacylglycerol obtained by incubation of a microbe being able to produce fat/oil where a polyunsaturated fatty acid is a constituting fatty acid and then it is fractionated by distillation.

26. A fat/oil in which triglyceride and diacylglycerol in a neutral lipid are not less than 95% and not more than 5%, respectively which is prepared in such a manner that fat/oil is extracted from microbial cells containing diacylglycerol and triacylglycerol obtained by incubation of a microbe being able to produce fat/oil where a polyunsaturated fatty acid is a constituting fatty acid and then it is fractionated by distillation.

27. A fat/oil where the polyunsaturated fatty acid in the fat/oil mentioned in claims 19 to 26 is a constituting fatty acid, **characterized in that**, the polyunsaturated fatty acid constituting the fat/oil is dihomo-γ-linolenic acid (20:3 ω6), arachidonic acid (20:4 ω6), 7, 10, 13, 16-docosatetraenoic acid (22:4 ω6), 4,7,10,13,16-docosapentaenoic acid (22:5 ω6), 6,9,12,15-octadecatetraenoic acid (18:4 ω3), 8,11,14,17-eicosatetraenoic acid (20:4 ω3), eicosapentaenoic acid (20:5 ω3), 7, 10, 13, 16, 19-docosapentaenoic acid (22:5 ω3), 4,7,10,13,16,19-docosahexaenoic acid (22:6 ω3), 6,9-octadecadienoic acid (18:2 ω9) , 8,11-eicosadienoic acid (20:2 ω9) or 5,8,11-eicosatrienoic acid (mead acid: 20:3 ω9) or a combination there of.

28. A food composition, an animal feeds composition, pet food composition, a material for chemical products, a drug and a cosmetic composition in which the fat/oil mentioned in any of claims 19 to 27 is contained, is used as a material or is chemically modified.
